# EUROPEAN PATENT APPLICATION

(11) **EP 1 394 240 A1**
(43) Date of publication of application: **03.03.2004**
(21) Application number: 03254694.7
(22) Date of filing: 28.07.2003
(51) Int. Cl.: C10M 135/14, C07D 487/08, C07D 209/52

(54) **Bicyclic thioamides as additives for lubricating oils**

(30) Priority: 07.08.2002 US 401541 P
(71) Applicant: ROHM AND HAAS COMPANY, Philadelphia, Pennsylvania 19106-2399 (US)
(72) Inventor: Mukkamala, Ravindranath, Lansdale Pennsylvania 19446 (US)
(74) Representative: Kent, Venetia Katherine

(57) **Abstract**

A composition comprising: (a) from 0.1% to 20% of at least one bicyclic thioamide of formula I: 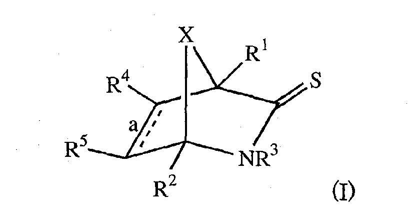 wherein X is O, S, NR⁶ or CR⁷R⁸; R¹, R², R⁴, R⁵, R⁷ and R⁸ independently are hydrogen, alkyl, alkenyl, aryl or aralkyl; R³ and R⁶ independently are hydrogen, alkyl, alkenyl, aryl, aralkyl, alkanoyl or aroyl; and bond "a" is a single or double bond; and (b) a lubricating oil.

## Description

### Background

This invention relates generally to bicyclic thioamides useful as additives for lubricating oils.

Zinc dialkyldithiophosphates (ZDDP) are widely used as lubricant additives. The principal disadvantages of these compounds are that an ash residue is produced by the zinc as the additive is consumed, and that phosphorus is known to affect the efficiency of catalytic converters in motor vehicles, thereby causing emissions problems. Cyclic compounds useful as lubricant additives are disclosed in U.S. Patent No. 6,187,722. However, the compounds disclosed therein are imidazolidinethiones which are not within the scope of the present invention.

The problem addressed by this invention is to find additional non-metallic, non-phosphorus-containing oil-soluble additives for lubricating oils.

### Statement of Invention

The present invention is directed to a composition comprising:
(a) from 0.1% to 20% of at least one bicyclic thioamide of formula I: 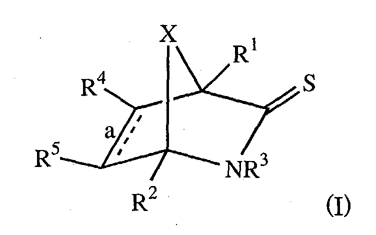 wherein X is O, S, NR⁶ or CR⁷R⁸; R¹, R², R⁴, R⁵, R⁷ and R⁸ independently are hydrogen, alkyl, alkenyl, aryl or aralkyl; R³ and R⁶ independently are hydrogen, alkyl, alkenyl, aryl, aralkyl, alkanoyl or aroyl; and bond "a" is a single or double bond; and
(b) a lubricating oil.

The present invention is further directed to a method for improving the anti-wear characteristics of a lubricating oil by adding from 0.1% to 20% of a compound of formula I.

The present invention is further directed to a lubricating oil composition containing the reaction product of a compound of formula (I) with an imine; an unsaturated carboxylic acid or ester; an isocyanate or isothiocyanate; or an alkyl, alkenyl or aralkyl group bearing a leaving group.

The present invention is further directed to a compound having formula (IV), as depicted herein, and a composition containing the compound and a lubricating oil.

### Detailed Description

All percentages are weight percentages based on the entire composition described, unless specified otherwise. An "alkyl" group is a saturated hydrocarbyl group having from one to twenty-two carbon atoms in a linear, branched or cyclic arrangement, and having from 0 to 2 oxygen, nitrogen or sulfur atoms. Substitution on alkyl groups of one or more halo, hydroxy, alkoxy, alkanoyl or amido groups is permitted; alkoxy, alkanoyl and amido groups may in turn be substituted by one or more halo substituents. In one preferred embodiment, alkyl groups contain from two to twelve carbon atoms and from 0 to 1 oxygen, nitrogen or sulfur atoms; in another preferred embodiment, alkyl groups contain from 4 to 22 carbon atoms; in another preferred embodiment, alkyl groups contain no heteroatoms. An "alkenyl" group is an "alkyl" group in which at least one carbon-carbon single bond has been replaced with a double bond. An "aryl" group is a substituent derived from an aromatic compound, including heterocyclic aromatic compounds having heteroatoms chosen from among nitrogen, oxygen and sulfur. An aryl group has a total of from five to twenty ring atoms, and has one or more rings which are separate or fused. Substitution on aryl groups of one or more halo, alkyl, alkenyl, hydroxy, alkoxy, alkanoyl or amido groups is permitted, with substitution by one or more halo groups being possible on alkyl, alkenyl, alkoxy, alkanoyl or amido groups. An "aralkyl" group is an "alkyl" group substituted by an "aryl" group. A "lubricating oil" is a natural or synthetic oil, or a mixture thereof, having suitable viscosity for use as a lubricant, e.g., as crankcase oil in an internal combustion engine, automatic transmission fluid, turbine lubricant, gear lubricant, compressor lubricant, metal-working lubricant, hydraulic fluid, etc.

Preferably, at least one of R¹, R², R³, R⁴, R⁵, R⁶, R⁷ and R⁸ is alkyl, alkenyl, aryl or aralkyl; more preferably at least two of R¹, R², R³, R⁴, R⁵, R⁶, R⁷ and R⁸ are alkyl, alkenyl, aryl or aralkyl. In one embodiment of the invention at least one of R¹, R², R³, R⁴, R⁵, R⁶, R⁷ and R⁸ is aryl, aralkyl, C₄-C₂₂ alkyl or C₄-C₂₂ alkenyl; more preferably at least one of R¹, R², R³, R⁴, R⁵, R⁶, R⁷ and R⁸ is C₄-C₂₂ alkyl or C₄-C₂₂ alkenyl; and most preferably at least one of R¹, R², R³, R⁴, R⁵, R⁶, R⁷ and R⁸ is C₆-C₂₂ alkyl. Preferably, X is NR⁶. The composition of the present invention includes at least one compound of formula (I). In one embodiment of the invention, the composition contains at least two compounds of formula (I), and more preferably contains at least three compounds of formula (I).

In one embodiment of the invention, bond "a" is a double bond, and the compound of formula (I) can be represented by formula (II) 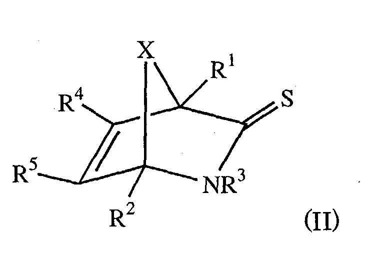 In one embodiment of the invention, a compound of formula (II) is prepared, for example, by the cycloaddition reaction of an isothiocyanate and a substituted or unsubstituted furan, thiophene, pyrrole or cyclopentadiene, as shown below in Scheme 1: 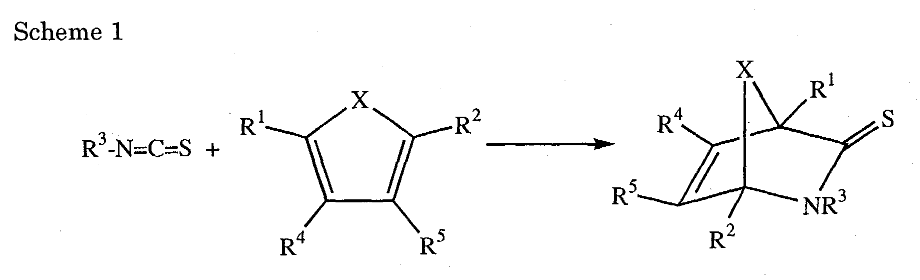 Preferably, R³ is C₆-C₂₂ alkyl, C₆-C₂₂ alkenyl, aryl or aralkyl; more preferably R³ is C₆-C₂₂ alkyl or aryl; most preferably R³ is C₈-C₂₂ alkyl. Preferably, R¹, R², R³, R⁴, R⁵, R⁶, R⁷ and R⁸ contain no heteroatoms. Preferably, at least one of R¹, R², R³, R⁴ and R⁵ is aryl, aralkyl, C₄-C₂₂ alkyl or C₄-C₂₂ alkenyl; more preferably C₆-C₂₂ alkyl or C₆-C₂₂ alkenyl; and most preferably C₁₀-C₂₂ alkyl. Preferably, X is NR⁶.

In one embodiment of the invention, bond "a" is a single bond, X is NR⁶, and the compound of formula (I) can be represented by formula (III). 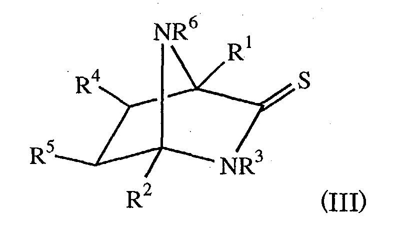 In one embodiment, a compound of formula (III) in which R³ and R⁶ are hydrogen, and R⁴ and R⁵ are hydrogen, is prepared from a 1,4-diketone, HCN and ammonium sulfide, in a two-step process, as shown below in Scheme 2:

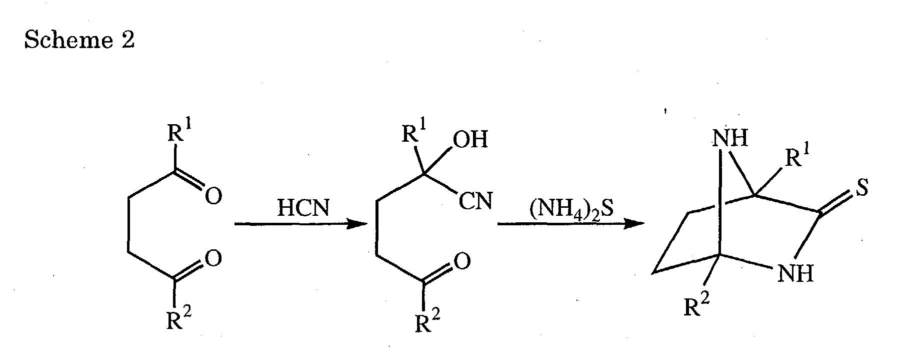

The NH amino group of the product optionally is further functionalized by reaction with alkyl, alkenyl, aralkyl, alkanoyl or aroyl groups having suitable leaving groups capable of being displaced by the NH group. For example, suitable leaving groups include iodide, bromide, chloride, tosylate, mesylate and triflate.

In a preferred embodiment of the invention, the thioamide group or a primary or secondary amino group of the compound of formula (I) is further functionalized by reaction with imines of formula R⁹N=CR¹⁰R¹¹; unsaturated carboxylic acids or esters of formula CHR¹²=CR¹³COOR¹⁴; isocyanates, R¹⁵NCO, or isothiocyanates, R¹⁵NCS; or R¹⁶Y; wherein R¹⁰, R¹¹ and R¹⁴ independently are hydrogen, alkyl, alkenyl, aryl or aralkyl; R¹⁶ is alkyl, alkenyl or aralkyl; R¹² and R¹³ independently are hydrogen or C₁-C₄ alkyl; R⁹ and R¹⁵ independently are alkyl, alkenyl, aryl or aralkyl; and Y is a suitable leaving group capable of being displaced by the nitrogen or the sulfur of a thioamide group, or by a primary or secondary amine nitrogen. For example, suitable leaving groups include iodide, bromide, chloride, tosylate, mesylate or triflate. Functionalization of the thioamide group can occur on the thioamide nitrogen or the thioamide sulfur, provided of course that the thioamide nitrogen bears at least one hydrogen. The product also can be a mixture of the compound functionalized on the nitrogen and the one functionalized on the sulfur. In compounds also having a primary or secondary amino group, functionalization also may occur solely or partially on the amino group.

Preferably, R¹² and R¹³ independently are hydrogen or methyl. Preferably, the compound of formula CHR¹²=CR¹³COOR¹⁴ is an alkyl or aralkyl acrylate having R¹²=R¹³=H and R¹⁴=alkyl or aralkyl; or a methacrylate ester having R¹²=H and R¹³=CH₃; or a crotonate ester having R¹³=H and R¹²=CH₃. Preferably, R¹² is hydrogen. Preferably, R¹⁴ is alkyl or aralkyl, most preferably C₄-C₂₂ alkyl. Preferably, R¹⁵ is aryl, aralkyl or alkyl; most preferably C₈-C₂₂ alkyl. Preferably, R⁹ is C₁₂-C₂₂ alkyl. In one embodiment, R⁹ is derived from an unsubstituted C₁₆-C₂₂ alkyl amine, R⁹NH₂, preferably one which is an oil-soluble amine. In one embodiment, the alkyl amine is a tertiary alkyl primary amine, i.e., a primary amine in which the alkyl group is attached to the amino group through a tertiary carbon. Examples of commercially available tertiary alkyl primary amines are the Primene^{TM} amines available from Rohm and Haas Company, Philadelphia, PA. Preferably, R¹⁰ and R¹¹ independently are alkyl or hydrogen. In a preferred embodiment of the invention, R⁹N=CR¹⁰R¹¹ is a formaldehyde imine, R⁹N=CH₂. Preferably, R¹⁶ is C₄-C₂₂ alkyl, C₄-C₂₂ alkenyl or aralkyl; more preferably C₆-C₂₂ alkyl or C₆-C₂₂ alkenyl; and most preferably C₈-C₂₂ alkyl.

As an example, the reaction of the compound of formula (I) in which X is CR⁷R⁸, R³ is hydrogen, and none of R¹, R², R⁴, R⁵, R⁷ and R⁸ contains a primary or secondary amine nitrogen, with an imine and an acrylate is illustrated below in Scheme 3.

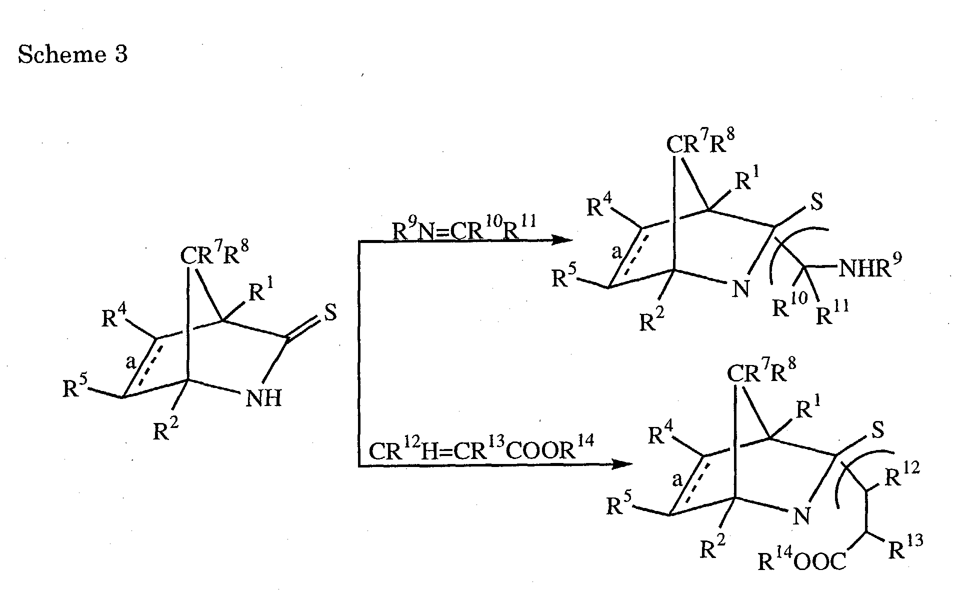

The compound resulting from functionalization of the thioamide group, or an NR⁶ group, with R⁶=H, in the compound of formula (I) in which X is CR⁷R⁸, O, S or NR⁶, wherein R⁶ is B³, alkyl, alkenyl, aryl or aralkyl; R³ is hydrogen; and none of R¹, R², R⁴, R⁵, R⁶, R⁷ and R⁸ contains a primary or secondary amine nitrogen, can be represented by formula (IV). 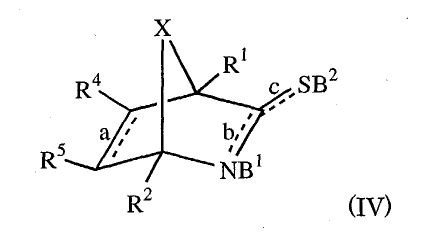 wherein bonds b and c are single or double bonds, provided that one of b and c is a single bond and the other is a double bond; at least one of B¹, B² and B³ is -CR¹²H-CR¹³H-COOR¹⁴, -CR¹⁰R¹¹NHR⁹, -C(O)NHR¹⁵, -C(S)NHR¹⁵ or R¹⁶; when one of B¹ and B² is -CR¹²H-CR¹³H-COOOR¹⁴, -CR¹⁰R¹¹NHR⁹, -C(O)NHR¹⁵, -C(S)NHR¹⁵ or R¹⁶, then the other is absent. When B¹ is present, bond "c" is a double bond; and when B² is present, bond "b" is a double bond. Preferably, at least one of B¹, B² and B³ is -CR¹²H-CR¹³H-COOR¹⁴, -CR¹⁰R¹¹NHR⁹, -C(O)NHR¹⁵ or -C(S)NHR¹⁵. Analogous structures exist for the compounds resulting from functionalization of compounds having a primary or secondary nitrogen atom as part of an "R" substituent, in which functionalization may occur on the primary or secondary nitrogen atom, as well as on the NR⁶ group if R⁶=H, or the thioamide nitrogen or sulfur.

Preferably, the compound(s) of formula (I) is present in a lubricating oil in a total amount of at least 0.2%, more preferably at least 0.3%, and most preferably at least 0.4%. Preferably, the compound(s) of formula (I) is present in a lubricating oil in a total amount no greater than 10%, more preferably no greater than 5%, and most preferably no greater than 2%. Preferably, the compounds are soluble at the aforementioned levels.

Optionally, other additives typically used in lubricating oils are present in the composition. Such additives include, but are not limited to, other anti-wear additives, anti-corrosion additives, dispersants, detergents, antioxidants, antifoamants, friction modifiers, seal swell agents, demulsifiers, viscosity index improvers and pour point depressants. Other anti-wear additives that can be used in combination with the compound of formula (I) include the commercial products known as ZDDP, which are zinc dialkyldithiophosphates. In addition to improving the anti-wear characteristics of lubricating oils, the compound of formula (I) typically also improves anti-corrosion characteristics and functions as an anti-oxidant.

## Claims

1. A composition comprising:
(a) from 0.1% to 20% of at least one bicyclic thioamide of formula I: 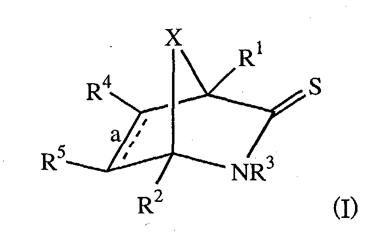 wherein X is O, S, NR⁶ or CR⁷R⁸; R¹, R², R⁴, R⁵, R⁷ and R⁸ independently are hydrogen, alkyl, alkenyl, aryl or aralkyl; R³ and R⁶ independently are hydrogen, alkyl, alkenyl, aryl, aralkyl, alkanoyl or aroyl; and bond "a" is a single or double bond; and
(b) a lubricating oil.

2. The composition of claim 1 in which at least one of R¹, R², R³, R⁴, R⁵, R⁶, R⁷ and R⁸ is aryl, aralkyl, C₄-C₂₂ alkyl or C₄-C₂₂ alkenyl.

3. The composition of claim 2 in which bond "a" is a double bond.

4. The composition of claim 3 in which R³ is C₆-C₂₂ alkyl, C₆-C₂₂ alkenyl, aryl or aralkyl.

5. The composition of claim 4 in which X is NR⁶.

6. The composition of claim 2 in which bond "a" is a single bond.

7. The composition of claim 6 in which X is NR⁶.

8. A composition comprising:
(a) a reaction product of a compound of formula (I) with an imine; an unsaturated carboxylic acid or ester; an isocyanate or isothiocyanate; or an alkyl, alkenyl or aralkyl group bearing a leaving group; and
(b) a lubricating oil.

9. A compound of formula (IV) 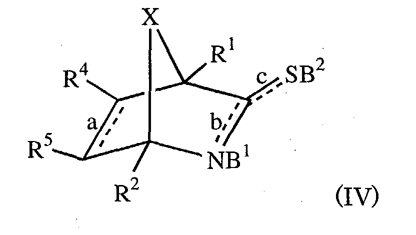 wherein X is O, S, NR⁶ or CR⁷R⁸; R¹, R², R⁴, R⁵, R⁷ and R⁸ independently are hydrogen, alkyl, alkenyl, aryl or aralkyl; R⁶ is B³, hydrogen, alkyl, alkenyl, aryl, aralkyl, alkanoyl or aroyl; bond "a" is a single or a double bond; bonds "b" and "c" are single or double bonds, provided that one of b and c is a single bond and the other is a double bond;
B¹, B² and B³ independently are -CR¹²H-CR¹³H-COOR¹⁴, -CR¹⁰R¹¹NHR⁹, -C(O)NHR¹⁵, -C(S)NHR¹⁵ or R¹⁶; provided that at least one of B¹, B² and B³ is -CR¹²H-CR¹³H-COOR¹⁴, -CR¹⁰R¹¹NHR⁹, -C(O)NHR¹⁵, -C(S)NHR¹⁵ or R¹⁶; and provided that when one of B¹ and B² is -CR¹²H-CR¹³H-COOR¹⁴, -CR¹⁰R¹¹NHR⁹,-C(O)NHR¹⁵, -C(S)NHR¹⁵ or R¹⁶, then the other of B¹ and B² is absent;
R¹⁰, R¹¹ and R¹⁴ independently are hydrogen, alkyl, alkenyl, aryl or aralkyl; R¹⁶ is alkyl, alkenyl or aralkyl; R¹² and R¹³ independently are hydrogen or C₁-C₄ alkyl; R⁹ and R¹⁵ independently are alkyl, alkenyl, aryl or aralkyl.

10. The compound of claim 9 in which at least one of B¹, B² and B³ is -CR¹²H-CR¹³H-COOR¹⁴, -CR¹⁰R¹¹NHR⁹, -C(O)NHR¹⁵ or -C(S)NHR¹⁵.
